# EUROPÄISCHE PATENTSCHRIFT

(11) **EP 4 370 578 B1**
(45) Veröffentlichungstag und Bekanntmachung des Hinweises auf die Patenterteilung: **23.04.2025**
(21) Anmeldenummer: 22743803.3
(22) Anmeldetag: 04.07.2022
(51) Int. Cl.: C08G 59/50, C09D 163/00, C09J 163/00

(54) **BESCHLEUNIGER FÜR EPOXIDHARZE**
ACCELERATOR FOR EPOXIDE RESINS
ACCÉLÉRATEUR POUR RÉSINES ÉPOXY

(30) Priorität: 12.07.2021 EP 21185057
(43) Veröffentlichungstag der Anmeldung: 22.05.2024
(73) Patentinhaber: Sika Technology AG, 6340 Baar (CH)
(72) Erfinder: KASEMI, Edis, 8046 Zürich (CH); BURCKHARDT, Urs, 8049 Zürich (CH); KRAMER, Andreas, 8008 Zürich (CH); STADELMANN, Ursula, 8046 Zürich (CH)
(74) Vertreter: Sika Patent Attorneys
(86) Internationale Anmeldenummer: PCT/EP2022/068434
(87) Internationale Veröffentlichungsnummer: WO 2023/285188

(56) Entgegenhaltungen:
- EP-A1- 3 568 390
- WO-A1-2015/124792

## Beschreibung

### Technisches Gebiet

Die Erfindung betrifft Amine und deren Verwendung zum Aushärten von Epoxidharzen.

### Stand der Technik

Epoxidharz-Zusammensetzungen werden unter anderem als Klebstoffe oder Beschichtungen in der Bau- und Fertigungsindustrie vielfältig eingesetzt. Es ist vorteilhaft, wenn die Zusammensetzungen schnell und zuverlässig aushärten und damit nach der Applikation möglichst frühzeitig mechanisch belastbar werden. Für eine raschere Aushärtung werden oft sogenannte Beschleuniger eingesetzt, insbesondere bei kalthärtenden Systemen. Als gute Beschleuniger bekannt sind insbesondere Substanzen aus der Kategorie der Phenole. Viele der altbekannten Beschleuniger, wie insbesondere Nonylphenol oder tert.Butylphenol, sind heutzutage aber kaum mehr einsetzbar, da sie als solche toxisch sind, bei der Aushärtung nicht ins Polymernetzwerk eingebaut werden und somit zu Emissionen, Kontaminationen oder Verfärbungen führen können. Am effektivsten als Beschleuniger für kalthärtende Systeme ist 2,4,6-Tris(dimethylaminomethyl)phenol. Es verströmt aber einen starken, unangenehmen Amingeruch. Weitere bekannte Beschleuniger sind Säuren wie insbesondere Salicylsäure. Sie verursachen zwar kaum Gerüche, bewirken aber eine starke Viskositätserhöhung und ihre beschleunigende Wirkung ist oft ungenügend.

Besonders vorteilhaft sind Beschleuniger, welche bei der Aushärtung ins Polymernetzwerk eingebaut werden. Aus dem Stand der Technik sind Aminhärter bekannt, welche zusätzlich als Beschleuniger wirken und bei der Aushärtung ins Polymernetzwerk eingebaut werden, beispielsweise 3-Dimethylaminopropylamin (DMAPA), 3-(3-(Dimethylamino)propylamino)propylamin (DMAPAPA) oder 2,4,6-Tris((2-dimethylaminopropyl)aminomethyl)phenol. Alle drei verursachen aber deutliche Blushing-Effekte bei kühlen Umgebungstemperaturen und flächiger Anwendung, und ihre beschleunigende Wirkung ist für viele Anwendungen noch verbesserungsfähig.

Alkylierte Amine sind als Härter für Epoxidharz-Beschichtungen bekannt, beispielsweise aus EP 3,180,383 oder EP 3,344,677. Sie zeigen eine zuverlässige Aushärtung bei Umgebungstemperaturen und wenig Neigung zu Blushing-Effekten bei flächiger Anwendung. Die damit erreichten Aushärtegeschwindigkeiten sind aber noch verbesserungsfähig.

Auch die WO 2015/124792 A1 beschäftigt sich mit Blushing-Effekten im Kontext von Aminhärtern für Epoxidharze.

### Darstellung der Erfindung

Aufgabe der vorliegenden Erfindung ist es daher, einen geruchsarmen Beschleuniger zum Aushärten von Epoxidharzen zur Verfügung zu stellen, welcher eine besonders schnelle Aushärtung ermöglicht, keine Viskositätserhöhung bewirkt und ins ausgehärtete Polymernetzwerk eingebaut wird.

Überraschenderweise löst ein Amin der Formel (I) wie in Anspruch 1 beschrieben diese Aufgabe. Das Amin der Formel (I) ist in einem einfachen Prozess durch reduktive Alkylierung eines Dimethylamins mit einem eine Formyl- oder Acetylgruppe tragenden Benzonitril und Wasserstoff herstellbar. Es ist geruchsarm, niedrigviskos und zeigt ausgezeichnete Eigenschaften als Härter und Beschleuniger. Es ermöglicht eine überraschend schnelle und störungsfreie Aushärtung und hohe Endhärten, wobei auch bei flächiger Anwendung und kalten Temperaturen schöne, fehlerfreie Oberflächen mit hohem Glanz entstehen.

Das Amin der Formel (I) ist geeignet als Härter, Co-Härter und/oder einbaubaren Beschleuniger für Epoxidharz-Produkte wie insbesondere Beschichtungen, Klebstoffe, Vergussmassen, Formkörper oder Matrixharze für Verbundwerkstoffe. Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist die Verwendung eines Amins der Formel (I) zum Aushärten von Epoxidharzen, 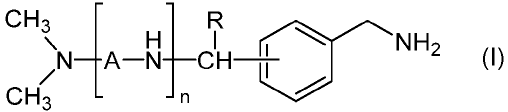 wobei n für 0 oder 1 oder 2 steht, R für H oder Methyl steht und A für einen Alkylen-Rest mit 2 bis 6 C-Atomen steht.

Mit "Poly" beginnende Substanznamen wie Polyamin oder Polyepoxid bezeichnen Substanzen, die formal zwei oder mehr der in ihrem Namen vorkommenden funktionellen Gruppen pro Molekül enthalten.

Als "primäre Aminogruppe" wird eine Aminogruppe bezeichnet, die an einen einzigen organischen Rest gebunden ist und zwei Wasserstoffatome trägt; als "sekundäre Aminogruppe" wird eine Aminogruppe bezeichnet, die an zwei organische Reste, welche auch gemeinsam Teil eines Rings sein können, gebunden ist und ein Wasserstoffatom trägt; und als "tertiäre Aminogruppe" wird eine Aminogruppe bezeichnet, die an drei organische Reste, welche auch zu zweit oder zu dritt Teil eines oder mehrerer Ringe sein können, gebunden ist und kein Wasserstoffatom trägt.

Als "Aminwasserstoff" werden die Wasserstoffatome von primären und sekundären Amingruppen bezeichnet.

Als "Aminwasserstoff-Equivalentgewicht" wird die Masse eines Amins oder einer Amin-haltigen Zusammensetzung, die ein Molequivalent Aminwasserstoff enthält, bezeichnet. Es wird angegeben in der Masseinheit "g/eq".

Als "Epoxid-Equivalentgewicht" wird die Masse einer Epoxidgruppen-haltigen Verbindung oder Zusammensetzung bezeichnet, die ein Mol-Equivalent Epoxidgruppen enthält. Es wird angegeben in der Masseinheit "g/eq".

Als "Verdünner" wird eine in einem Epoxidharz lösliche und dessen Viskosität senkende Substanz bezeichnet, welche bei der Aushärtung chemisch nicht in das Epoxidharz-Polymer eingebunden wird.

Als "Molekulargewicht" wird die molare Masse (in Gramm pro Mol) eines Moleküls bezeichnet. Als "mittleres Molekulargewicht" wird das Zahlenmittel Mₙ einer polydispersen Mischung von oligomeren oder polymeren Molekülen bezeichnet, welches üblicherweise mittels Gelpermeationschromatographie (GPC) gegen Polystyrol als Standard bestimmt wird.

Als "Topfzeit" wird die maximale Zeitspanne ab dem Mischen der Komponenten und der Applikation einer Epoxidharz-Zusammensetzung bezeichnet, in der die vermischte Zusammensetzung in einem ausreichend fliessfähigen Zustand ist und die Substratoberflächen gut benetzen kann.

Als "Gelierzeit" wird die Zeitspanne ab dem Mischen der Komponenten einer Epoxidharz-Zusammensetzung bis zu deren Gelieren bezeichnet.

Als "Raumtemperatur" wird eine Temperatur von 23 °C bezeichnet.

Alle im Dokument erwähnten Industriestandards und Normen beziehen sich auf die zum Zeitpunkt der Einreichung der Erstanmeldung gültigen Fassungen, sofern nicht anders angegeben.

Gewichtsprozente (Gewichts-%) bezeichnen Massenanteile eines Bestandteils einer Zusammensetzung bezogen auf die gesamte Zusammensetzung, falls nichts anderes angegeben ist. Die Begriffe "Masse" und "Gewicht" werden im vorliegenden Dokument synonym benutzt.

Bevorzugt steht R für H. Diese Amine der Formel (I) ermöglichen eine besonders schnelle Aushärtung.

Bevorzugt steht A für 1,2-Ethylen, 1,2-Propylen, 1,3-Propylen, 1,4-Butylen oder 1,5-Pentylen.

Besonders bevorzugt steht A für 1,2-Ethylen oder 1,3-Propylen, insbesondere für 1,3-Propylen.

Bevorzugt steht n für 0 oder 1. Besonders bevorzugt steht n für 1.

Bevorzugt ist das Amin der Formel (I) ausgewählt aus der Gruppe bestehend aus N,N-Dimethyl-1,3-bis(aminomethyl)benzol, N-(2-Dimethylaminoethyl)-1,3-bis(aminomethyl)benzol, N-(3-Dimethylaminopropyl)-1,3-bis(aminomethyl)benzol, N,N-Dimethyl-1,4-bis(aminomethyl)benzol, N-(2-Dimethylaminoethyl)-1,4-bis(aminomethyl)benzol, N-(3-Dimethylaminopropyl)-1,4-bis(aminomethyl)benzol, N,N-Dimethyl-1,2-bis(aminomethyl)benzol, N-(2-Dimethylaminoethyl)-1,2-bis(aminomethyl)benzol und N-(3-Dimethylaminopropyl)-1,2-bis(aminomethyl)benzol.

Bevorzugt stehen die Substituenten am Benzolring in 1,3- oder in 1,4-Stellung, insbesondere in 1,3-Stellung.

Davon bevorzugt ist N-(3-Dimethylaminopropyl)-1,3-bis(aminomethyl)benzol oder N-(3-Dimethylaminopropyl)-1,4-bis(aminomethyl)benzol.

Das Amin der Formel (I) ist bevorzugt Bestandteil eines Reaktionsprodukts erhalten aus der reduktiven Alkylierung von mindestens einem Amin der Formel (II) mit mindestens einem Benzonitril der Formel (III) und Wasserstoff, 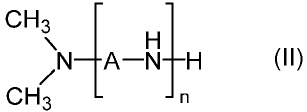 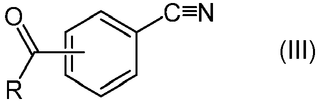 wobei n, R und A die bereits genannten Bedeutungen aufweisen.

Als Amin der Formel (II) bevorzugt ist Dimethylamin, 2-Dimethylaminoethylamin, 3-Dimethylamino-2-propylamin, 3-Dimethylaminopropylamin (DMAPA), 4-Dimethylaminobutylamin, 5-Dimethylaminopentylamin oder 3-(3-(Dimethylamino)propyl-amino)propylamin (DMAPAPA).

Besonders bevorzugt ist Dimethylamin, 2-Dimethylaminoethylamin oder 3-Dimethylaminopropylamin (DMAPA), insbesondere DMAPA.

Als Benzonitril der Formel (III) geeignet ist 2-Formylbenzonitril, 3-Formylbenzonitril oder 4-Formylbenzonitril, 2-Acetylbenzonitril, 3-Acetylbenzonitril oder 4-Acetylbenzonitril. Bevorzugt ist 2-Formylbenzonitril, 3-Formylbenzonitril oder 4-Formylbenzonitril, insbesondere 3-Formylbenzonitril oder 4-Formylbenzonitril, am meisten bevorzugt ist 3-Formylbenzonitril.

Das Amin der Formel (II) und das Benzonitril der Formel (III) werden bevorzugt ungefähr stöchiometrisch eingesetzt, insbesondere im Molverhältnis zwischen dem Amin der Formel (II) und dem Benzonitril der Formel (III) von 0.9/1 bis 1.1/1, bevorzugt 0.9/1 bis 1.0/1.

Die reduktive Alkylierung kann direkt mit molekularem Wasserstoff oder indirekt durch Wasserstoff- oder Hydrid-Transfer von anderen Reagenzien wie Ameisensäure oder LiAlH₄ erfolgen. Bevorzugt wird molekularer Wasserstoff verwendet.

Die Bedingungen bei der Umsetzung werden vorteilhaft so gewählt, dass die Nitrilgruppe ebenfalls hydriert wird und der Benzolring nicht hydriert wird. Bevorzugt wird die Umsetzung bei einer Temperatur von 40 bis 120 °C und in Anwesenheit eines geeigneten Katalysators durchgeführt. Als Katalysator bevorzugt sind Palladium auf Kohle (Pd/C), Platin auf Kohle (Pt/C), Adams-Katalysator oder Raney-Nickel, insbesondere Palladium auf Kohle oder Raney-Nickel.

Bei Verwendung von molekularem Wasserstoff wird bevorzugt in einer Druckapparatur bei einem Wasserstoff-Druck von 5 bis 150 bar, insbesondere 10 bis 120 bar, gearbeitet.

Bevorzugt werden die flüchtigen Anteile, insbesondere ein gegebenenfalls vorhandenes Lösemittel und Wasser, nach der Umsetzung aus dem Reaktionsprodukt entfernt, insbesondere mittels Destillation oder Stripping.

Das Reaktionsprodukt kann weiter gereinigt werden, insbesondere durch Destillation. Dadurch wird ein Reaktionsprodukt mit einem besonders hohen Gehalt an Amin der Formel (I) erhalten.

Bevorzugt enthält das Reaktionsprodukt einen Gehalt an Amin der Formel (I) von mindestens 70 Gewichts-%, besonders bevorzugt mindestens 80 Gewichts-%, insbesondere mindestens 90 Gewichts-%.

Das Reaktionsprodukt kann als Nebenprodukte insbesondere folgende Verbindungen enthalten:
- Verbindungen mit Imin- und/oder Nitrilgruppen aus der unvollständigen Hydrierung der Zwischenstufen, im Fall von n=1 beispielsweise der Formel 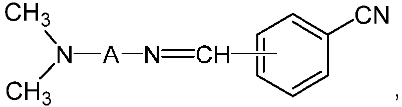
- Amine der Formel 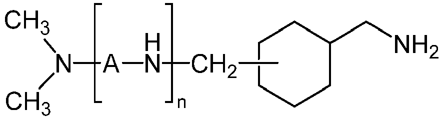 mit hydriertem Benzolring,
- Aminoalkohole der Formel 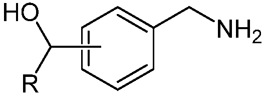 aus der Hydrierung von nicht umgesetztem Benzonitril der Formel (III),
- mehrfach alkylierte Amine, wie beispielsweise im Fall von DMAPA und 3-Formylbenzonitril das Folgende:

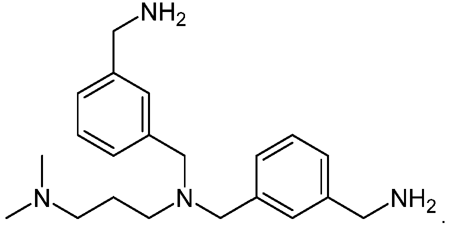

Das Amin der Formel (I) ist geruchsarm und niedrigviskos.

Das Amin der Formel (I), insbesondere in Form des beschriebenen Reaktionsprodukts, wird verwendet zum Aushärten von Epoxidharzen.

Geeignete Epoxidharze sind insbesondere Glycidylether von handelsüblichen ein-oder mehrfunktionellen Alkoholen oder Phenolen, insbesondere die in diesem Dokument genannten Epoxidharze.

Bevorzugt werden das Amin der Formel (I), das Epoxidharz und gegebenenfalls weitere Substanzen miteinander vermischt, wobei das Verhältnis der gegenüber den Epoxidgruppen reaktiven Gruppen, insbesondere den Aminwasserstoffen des Amins der Formel (I), und den Epoxidgruppen bevorzugt im Bereich von 0.5 bis 1.5, insbesondere 0.7 bis 1.2, liegt.

Die Aushärtung kann bei Umgebungstemperatur, insbesondere im Bereich von 5 bis 40 °C, oder bei erhöhter Temperatur, insbesondere im Bereich von 40 bis 150 °C, bevorzugt 50 bis 120 °C, erfolgen.

Das Amin der Formel (I) ermöglicht eine überraschend schnelle und störungsfreie Aushärtung und hohe Endhärten, wobei auch bei flächiger Anwendung und kalten Temperaturen schöne, fehlerfreie Oberflächen mit hohem Glanz entstehen.

Das Amin der Formel (I) kann in Kombination mit weiteren Härtern verwendet werden.

Das Amin der Formel (I) kann in teilweise mit mindestens einem Epoxidharz oder Monoepoxid adduktierter Form eingesetzt werden, insbesondere mit einem Epoxidharz oder Monoepoxid mit einem mittleren Epoxid-Equivalentgewicht im Bereich von 150 bis 500 g/eq, bevorzugt 156 bis 250 g/eq.

Bevorzugt sind aminfunktionelle Addukte aus der Umsetzung von mindestens einem Amin der Formel (I) und mindestens einem Epoxidharz in einem stöchiometrischen Verhältnis im Bereich von 1 bis 10, bevorzugt 1 bis 5, mol Amin der Formel (I) pro Molequivalent Epoxidgruppen.

Ein weiterer Gegenstand der Erfindung ist ein Härter für Epoxidharze enthaltend mindestens ein Amin der Formel (I) und mindestens einen weiteren Bestandteil ausgewählt aus weiteren Aminen **A1,** Beschleunigern und Verdünnern.

Bevorzugt ist das weitere Amin **A1** kein Amin der Formel (I).

Der Härter enthält bevorzugt 1 bis 99 Gewichts-%, mehr bevorzugt 2 bis 90 Gewichts-%, mehr bevorzugt 3 bis 75 Gewichts-%, besonders bevorzugt 5 bis 50 Gewichts-%, insbesondere 7 bis 30 Gewichts-%, Amin der Formel (I), bezogen auf den gesamten Härter.

Der Härter ist bevorzugt nicht wasserbasiert. Er enthält bevorzugt weniger als 15 Gewichts-%, insbesondere weniger als 10 Gewichts-%, Wasser, bezogen auf den gesamten Härter. Ein solcher Härter ist besonders geeignet für nicht-wässrige Epoxidharz-Produkte.

Geeignete weitere Amine sind insbesondere Amine mit mindestens zwei, bevorzugt mindestens drei, Aminwasserstoffen, welche nicht der Formel (I) entsprechen.

Bevorzugt enthält der Härter mindestens ein weiteres Amin A1, welches mindestens drei aliphatische Aminwasserstoffe aufweist.

Dafür geeignet sind insbesondere N-Benzyl-1,2-ethandiamin, N-Benzyl-1,2-propandiamin, N-Benzyl-bis(aminomethyl)-1,3-benzol, N-(2-Phenylethyl)-bis(aminomethyl)-1,3-benzol, N-(2-Ethylhexyl)-bis(aminomethyl)-1,3-benzol, 2,2-Dimethyl-1,3-propandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2(4),4-Trimethyl-1,6-hexandiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- oder 1,4-Diaminocyclohexan, 1,3-Bis(aminomethyl)cyclohexan, 1,4-Bis(aminomethyl)cyclohexan, Bis(4-aminocyclohexyl)methan, Bis(4-amino-3-methylcyclohexyl)methan, Bis(4-amino-3-ethylcyclohexyl)methan, Bis(4-amino-3,5-dimethylcyclohexyl)methan, Bis(4-amino-3-ethyl-5-methylcyclohexyl)methan, 1-Amino-3-aminomethyl-3,5,5-trimethylcyclohexan (IPDA), 2(4)-Methyl-1,3-diaminocyclohexan, 2,5(2,6)-Bis(aminomethyl)-bicyclo[2.2.1]heptan (NBDA), 3(4),8(9)-Bis(aminomethyl)tricyclo[5.2.1.0^{2,6}]decan, 1,4-Diamino-2,2,6-trimethylcyclohexan (TMCDA), 1,8-Menthandiamin, 3,9-Bis(3-aminopropyl)-2,4,8,10-tetraoxaspiro[5.5]undecan, 1,3-Bis(aminomethyl)benzol (MXDA), 1,4-Bis(aminomethyl)benzol, Bis(2-aminoethyl)ether, 3,6-Dioxaoctan-1,8-diamin, 4,7-Dioxadecan-1,10-diamin, 4,7-Dioxadecan-2,9-diamin, 4,9-Dioxadode-can-1,12-diamin, 5,8-Dioxadodecan-3,10-diamin, 4,7,10-Trioxatridecan-1,13-diamin oder höhere Oligomere dieser Diamine, Bis(3-aminopropyl)polytetrahydro-furane oder andere Polytetrahydrofurandiamine, Polyoxyalkylendi- oder -triamine, insbesondere Polyoxypropylendiamine oder Polyoxypropylentriamine wie insbesondere Jeffamine^{®} D-230, Jeffamine^{®} D-400 oder Jeffamine^{®} T-403 (alle von Huntsman), Polyalkylenamine wie Diethylentriamin (DETA), Triethylentetramin (TETA), Tetraethylenpentamin (TEPA), Pentaethylenhexamin (PEHA), Dipropylentriamin (DPTA), N-(2-Aminoethyl)-1,3-propandiamin (N3-Amin), N,N'-Bis(3-aminopropyl)ethylendiamin (N4-Amin), N,N'-Bis(3-aminopropyl)-1,4-diaminobutan, N5-(3-Aminopropyl)-2-methyl-1,5-pentandiamin, N3-(3-Aminopentyl)-1,3-pentandiamin, N5-(3-Amino-1-ethylpropyl)-2-methyl-1,5-pentandiamin, N,N'-Bis(3-amino-1-ethylpropyl)-2-methyl-1,5-pentandiamin, 3-(2-Aminoethyl)aminopropylamin oder Bis(hexamethylen)triamin (BHMT), N-benzylierte Polyalkylenamine mit einer oder zwei Benzylgruppen, insbesondere benzyliertes DETA, TETA, N3-Amin, N4-Amin oder DPTA, N-Aminoethylpiperazin, 3-(3-(Dimethylamino)propylamino)propylamin (DMAPAPA), aminfunktionelle Addukte der genannten Amine mit Epoxiden, oder Mischungen aus zwei oder mehr dieser Amine.

Bevorzugt enthält der Härter mindestens ein weiteres Amin A1 ausgewählt aus der Gruppe bestehend aus N-Benzyl-1,2-ethandiamin, MPMD, TMD, 1,2-Diaminocyclohexan, 1,3-Bis(aminomethyl)cyclohexan, 1,4-Bis(aminomethyl)cyclohexan, Bis(4-aminocyclohexyl)methan, IPDA, 2(4)-Methyl-1,3-diaminocyclohexan, NBDA, MXDA, DETA, TETA, TEPA, N3-Amin, N4-Amin, DPTA, BHMT, DMAPAPA, Polyoxypropylendiaminen mit einem mittlerem Molekulargewicht Mₙ im Bereich von 200 bis 500 g/mol und Polyoxypropylentriaminen mit einem mittlerem Molekulargewicht Mₙ im Bereich von 300 bis 500 g/mol.

Davon bevorzugt ist N-Benzyl-1,2-ethandiamin. Damit werden Beschichtungen mit besonders schönen Oberflächen ermöglicht.

Davon bevorzugt ist weiterhin MXDA, 1,3-Bis(aminomethyl)cyclohexan oder 1,4-Bis(aminomethyl)cyclohexan. Damit wird eine besonders schnelle Aushärtung ermöglicht.

Davon bevorzugt ist weiterhin IPDA. Damit wird ein kostengünstiger Härter mit hoher Glasübergangstemperatur ermöglicht.

Davon bevorzugt ist weiterhin DETA, TETA, TEPA oder N4-Amin. Damit werden kostengünstige Klebstoffe mit besonders hoher Haftkraft ermöglicht.

Der Härter kann insbesondere mehr als ein weiteres Amin **A1** enthalten.

Geeignete Beschleuniger sind insbesondere Säuren oder zu Säuren hydrolysierbare Verbindungen, insbesondere organische Carbonsäuren wie Essigsäure, Benzoesäure, Salicylsäure, 2-Nitrobenzoesäure, Milchsäure, organische Sulfonsäuren wie Methansulfonsäure, p-Toluolsulfonsäure oder 4-Dodecylbenzolsulfonsäure, Sulfonsäureester, andere organische oder anorganische Säuren wie insbesondere Phosphorsäure, oder Mischungen der vorgenannten Säuren und Säureester; Nitrate wie insbesondere Calciumnitrat; tertiäre Amine wie insbesondere 1,4-Diazabicyclo[2.2.2]octan, Benzyldimethylamin, α-Methylbenzyldimethylamin, Triethanolamin, Dimethylaminopropylamin, Imidazole wie insbesondere N-Methylimidazol, N-Vinylimidazol oder 1,2-Dimethylimidazol, Salze solcher tertiärer Amine, quaternäre Ammoniumsalze, wie insbesondere Benzyltrimethylammoniumchlorid, Amidine wie insbesondere 1,8-Diazabicyclo[5.4.0]-undec-7-en, Guanidine wie insbesondere 1,1,3,3-Tetramethylguanidin, Phenole, insbesondere Bisphenole, Phenol-Harze oder Mannich-Basen wie insbesondere 2-(Dimethylaminomethyl)phenol, 2,4,6-Tris(dimethylaminomethyl)phenol oder Polymere aus Phenol, Formaldehyd und N,N-Dimethyl-1,3-propandiamin, Phosphite wie insbesondere Di- oder Triphenylphosphite, oder Mercaptogruppen aufweisende Verbindungen.

Bevorzugt sind Säuren, Nitrate, tertiäre Amine oder Mannich-Basen, insbesondere Salicylsäure, Calciumnitrat oder 2,4,6-Tris(dimethylaminomethyl)phenol, oder eine Kombination dieser Beschleuniger.

Geeignete Verdünner sind insbesondere n-Propanol, Isopropanol, n-Butanol, Isobutanol, tert. Butanol, 1-Pentanol, 2-Pentanol, 3-Pentanol, 2-Methyl-1-butanol, 3-Methyl-1-butanol, 3-Methyl-2-butanol n-Hexanol, 2-Ethylhexanol, Xylol, 2-Methoxyethanol, Dimethoxyethanol, 2-Ethoxyethanol, 2-Propoxyethanol, 2-Isopropoxyethanol, 2-Butoxyethanol, 2-Phenoxyethanol, 2-Benzyloxyethanol, Benzylalkohol, Ethylenglykol, Ethylenglykoldimethylether, Ethylenglykoldiethylether, Ethylenglykoldibutylether, Ethylenglykoldiphenylether, Diethylenglykol, Diethylenglykolmonomethylether, Diethylenglykolmonoethylether, Diethylenglykolmono-n-butylether, Diethylenglykoldimethylether, Diethylenglykoldiethylether, Diethylenglykoldi-n-butylylether, Propylenglykolbutylether, Propylenglykolphenylether, Dipropylenglykol, Dipropylenglykolmonomethylether, Dipropylenglykoldimethylether, Dipropylenglykoldi-n-butylether, 2,2,4-Trimethyl-1,3-pentandiolmonoisobutyrat, Diphenylmethan, Diisopropylnaphthalin, Erdölfraktionen wie zum Beispiel Solvesso^{®}-Typen (von Exxon), Alkylphenole wie tert. Butylphenol, Nonylphenol, Dodecylphenol, Cardanol, styrolisiertes Phenol, Bisphenole, aromatische Kohlenwasserstoffharze, insbesondere Phenolgruppen-haltige Typen, alkoxyliertes Phenol, insbesondere ethoxyliertes oder propoxyliertes Phenol, insbesondere 2-Phenoxyethanol, Adipate, Sebacate, Phthalate, Benzoate, organische Phosphor- oder Sulfonsäureester oder Sulfonamide.

Davon bevorzugt sind Verdünner mit einem Siedepunkt von mehr als 200 °C, insbesondere Benzylalkohol, styrolisiertem Phenol, ethoxyliertes Phenol, phenolgruppenhaltige aromatische Kohlenwasserstoffharze wie insbesondere die Novares^{®}-Typen LS 500, LX 200, LA 300 oder LA 700 (von Rütgers), Diisopropylnaphthalin oder Cardanol, insbesondere Benzylalkohol.

Phenolgruppen-haltige Verdünner zeigen auch eine Wirkung als Beschleuniger.

Der Härter kann weitere Bestandteile enthalten, insbesondere:
- weitere Addukte, insbesondere Addukte von MPMD oder 1,2-Ethandiamin oder 1,2-Propandiamin mit Kresylglycidylether oder aromatischen Epoxidharzen, bei welchen nicht umgesetztes MPMD, 1,2-Ethandiamin oder 1,2-Propandiamin nach der Umsetzung destillativ entfernt wurde,
- Monoamine wie insbesondere Benzylamin oder Furfurylamin,
- Polyamidoamine, insbesondere Umsetzungsprodukte aus einer ein- oder mehrwertigen Carbonsäure oder deren Ester oder Anhydrid, insbesondere einer Dimerfettsäure, mit einem im stöchiometrischen Überschuss eingesetzten Polyamin, insbesondere DETA oder TETA,
- Mannich-Basen, insbesondere Phenalkamine, also Umsetzungsprodukte von Phenolen, insbesondere Cardanol, mit Aldehyden, insbesondere Formaldehyd, und Polyaminen,
- aromatische Polyamine wie insbesondere 4,4'-, 2,4' und/oder 2,2'-Diaminodiphenylmethan, 2,4(6)-Toluoldiamin, 3,5-Dimethylthio-2,4(6)-toluoldiamin oder 3,5-Diethyl-2,4(6)-toluylendiamin,
- Mercaptogruppen aufweisende Verbindungen, insbesondere flüssige Mercaptan-terminierte Polysulfidpolymere, Mercaptan-terminierte Polyoxyalkylenether, Mercaptan-terminierte Polyoxyalkylen-Derivate, Polyester von Thiocarbonsäuren, 2,4,6-Trimercapto-1,3,5-triazin, Triethylenglykoldimercaptan oder Ethandithiol,
- oberflächenaktive Additive, insbesondere Entschäumer, Entlüfter, Netzmittel, Dispergiermittel oder Verlaufsmittel, oder
- Stabilisatoren, insbesondere Stabilisatoren gegen Oxidation, Wärme, Licht oder UV-Strahlung.

Ein weiterer Gegenstand der Erfindung ist eine Epoxidharz-Zusammensetzung umfassend
- eine Harz-Komponente enthaltend mindestens ein Epoxidharz und
- eine Härter-Komponente enthaltend mindestens ein Amin der Formel (I) oder einen Härter enthaltend mindestens ein Amin der Formel (I) wie vorgängig beschrieben.

Ein geeignetes Epoxidharz wird auf bekannte Art und Weise erhalten, insbesondere aus der Reaktion von Epichlorhydrin mit Polyolen, Polyphenolen oder Aminen.

Geeignete Epoxidharze sind insbesondere aromatische Epoxidharze, insbesondere die Glycidylether von:
- Bisphenol A, Bisphenol F oder Bisphenol A/F, wobei A für Aceton und F für Formaldehyd steht, welche als Edukte zur Herstellung dieser Bisphenole dienten. Im Fall von Bisphenol F können auch Stellungsisomere vorhanden sein, insbesondere abgeleitet von 2,4'- oder 2,2'-Hydroxyphenylmethan.
- Dihydroxybenzol-Derivaten wie Resorcin, Hydrochinon oder Brenzcatechin;
- weiteren Bisphenolen oder Polyphenolen wie Bis(4-hydroxy-3-methylphenyl)-methan, 2,2-Bis(4-hydroxy-3-methylphenyl)propan (Bisphenol-C), Bis(3,5-dimethyl-4-hydroxyphenyl)methan, 2,2-Bis(3,5-dimethyl-4-hydroxyphenyl)propan, 2,2-Bis(3,5-dibromo-4-hydroxyphenyl)propan, 2,2-Bis(4-hydroxy-3-tert.butylphenyl)propan, 2,2-Bis(4-hydroxyphenyl)butan (Bisphenol-B), 3,3-Bis(4-hydroxyphenyl)pentan, 3,4-Bis(4-hydroxyphenyl)hexan, 4,4-Bis(4-hydroxyphenyl)-heptan, 2,4-Bis(4-hydroxyphenyl)-2-methylbutan, 2,4-Bis(3,5-dimethyl-4-hydroxyphenyl)-2-methylbutan, 1,1-Bis(4-hydroxyphenyl)cyclohexan (Bisphenol-Z), 1,1-Bis(4-hydroxyphenyl)-3,3,5-trimethylcyclohexan (Bisphenol-TMC), 1,1-Bis(4-hydroxyphenyl)-1-phenylethan, 1,4-Bis[2-(4-hydroxyphenyl)-2-propyl]-benzol (Bisphenol-P), 1,3-Bis[2-(4-hydroxyphenyl)-2-propyl]benzol (Bisphenol-M), 4,4'-Dihydroxydiphenyl (DOD), 4,4'-Dihydroxybenzophenon, Bis(2-hydroxy-naphth-1-yl)methan, Bis(4-hydroxynaphth-1-yl)methan, 1,5-Dihydroxynaphthalin, Tris(4-hydroxyphenyl)methan, 1,1,2,2-Tetrakis(4-hydroxyphenyl)ethan, Bis(4-hydroxyphenyl)ether oder Bis(4-hydroxyphenyl)sulfon;
- Novolaken, welche insbesondere Kondensationsprodukte von Phenol oder Kresolen mit Formaldehyd bzw. Paraformaldehyd oder Acetaldehyd oder Crotonaldehyd oder Isobutyraldehyd oder 2-Ethylhexanal oder Benzaldehyd oder Furfural sind;
- aromatischen Aminen, wie Anilin, Toluidin, 4-Aminophenol, 4,4'-Methylendiphenyldiamin, 4,4'-Methylendiphenyldi-(N-methyl)amin, 4,4'-[1,4-Phenylen-bis(1-methylethyliden)]bisanilin (Bisanilin-P) oder 4,4'-[1,3-Phenylen-bis(1-methylethyliden)]bisanilin (Bisanilin-M).

Weitere geeignete Epoxidharze sind aliphatische oder cycloaliphatische Polyepoxide, insbesondere
- Glycidylether von gesättigten oder ungesättigten, verzweigten oder unverzweigten, zyklischen oder offenkettigen di-, tri- oder tetrafunktionellen C₂- bis C₃₀-Alkoholen, insbesondere Ethylenglykol, Propylenglykol, Butylenglykol, Hexandiol, Octandiol, Polypropylenglykolen, Dimethylolcyclohexan, Neopentylglykol, Dibromoneopentylglykol, Rizinusöl, Trimethylolpropan, Trimethylolethan, Pentaerythrol, Sorbit oder Glycerin, oder alkoxyliertes Glycerin oder alkoxyliertes Trimethylolpropan;
- ein hydriertes Bisphenol-A-, -F- oder -A/F-Flüssigharz, beziehungsweise die Glycidylisierungsprodukte von hydriertem Bisphenol-A, -F oder -A/F;
- ein N-Glycidylderivat von Amiden oder heterocyclischen Stickstoffbasen, wie Triglycidylcyanurat oder Triglycidylisocyanurat, oder Umsetzungsprodukte von Epichlorhydrin mit Hydantoin.

Bevorzugt ist das Epoxidharz ein Flüssigharz oder eine Mischung enthaltend zwei oder mehr Epoxid-Flüssigharze.

Als "Epoxid-Flüssigharz" wird ein technisches Polyepoxid mit einer Glasübergangstemperatur unterhalb von 25 °C bezeichnet.

Gegebenenfalls enthält die Harz-Komponente zusätzlich Anteile von Epoxid-Festharz.

Das Epoxidharz ist insbesondere ein Flüssigharz auf der Basis eines Bisphenols oder Novolaks, insbesondere mit einem mittleren Epoxid-Equivalentgewicht im Bereich von 156 bis 210 g/eq.

Besonders geeignet ist ein Bisphenol-A Diglycidylether und/oder Bisphenol-F-Diglycidylether, wie sie kommerziell beispielsweise von Olin, Huntsman oder Momentive erhältlich sind. Diese Flüssigharze weisen eine für Epoxidharze niedrige Viskosität auf und ermöglichen eine schnelle Aushärtung und hohe Härten. Sie können Anteile von Bisphenol A-Festharz oder Novolak-Epoxidharzen enthalten.

Weiterhin besonders geeignet sind Phenol-Formaldehyd Novolak-Glycidylether, insbesondere mit einer mittleren Funktionalität im Bereich von 2.3 bis 4, bevorzugt 2.5 bis 3. Sie können Anteile von weiteren Epoxidharzen enthalten, insbesondere Bisphenol-A Diglycidylether oder Bisphenol-F-Diglycidylether.

Die Harz-Komponente kann einen Reaktivverdünner enthalten.

Als Reaktivverdünner bevorzugt sind Epoxidgruppen-haltige Reaktivverdünner, insbesondere Butandioldiglycidylether, Hexandioldiglycidylether, Trimethylolpropandi- oder triglycidylether, Phenylglycidylether, Kresylglycidylether, Guaiacolglycidylether, 4-Methoxyphenylglycidylether, p-n-Butylphenylglycidylether, p-tert.Butylphenylglycidylether, 4-Nonylphenylglycidylether, 4-Dodecylphenylglycidylether, Cardanolglycidylether, Benzylglycidylether, Allylglycidylether, Butylglycidylether, Hexylglycidylether, 2-Ethylhexylglycidylether, oder Glycidylether von natürlichen Alkoholen wie insbesondere C₈- bis C₁₀- oder C₁₂- bis C₁₄- oder C₁₃-bis C₁₅-Alkylglycidylether.

Bevorzugt enthält die Epoxidharz-Zusammensetzung mindestens einen weiteren Bestandteil ausgewählt aus der Gruppe bestehend aus Verdünnern, Beschleunigern, Stabilisatoren, oberflächenaktiven Additiven, Füllstoffen und Pigmenten.

Als Verdünnern, Beschleuniger, Stabilisatoren und oberflächenaktive Additive geeignet sind insbesondere die bereits genannten.

Geeignete Füllstoffe sind insbesondere gemahlenes oder gefälltes Calciumcarbonat, welches gegebenenfalls mit Fettsäure, insbesondere Stearaten, beschichtet ist, Baryt (Schwerspat), Talk, Quarzmehl, Quarzsand, Siliciumcarbid, Eisenglimmer, Dolomit, Wollastonit, Kaolin, Mica (Kalium-Aluminium-Silikat), Molekularsiebe, Aluminiumoxid, Zinkoxid, Aluminium-dotiertes Zinkoxid, Aluminiumhydroxid, Magnesiumhydroxid, Kieselsäure, Zement, Gips, Flugasche, Russ, Graphit, Holzmehl, Metall-Pulver wie Aluminium, Kupfer, Eisen, Zink, Silber oder Stahl, PVC-Pulver oder Hohlkugeln. Davon bevorzugt sind Calciumcarbonat, Baryt, Quarzmehl, Talk, oder eine Kombination davon.

Geeignete Pigmente sind insbesondere Titandioxide, Eisenoxide, Chrom(III)oxide, Korrosionsschutzpigmente, organische Pigmente oder Russ, insbesondere Titandioxide.

Gegebenenfalls enthält die Epoxidharz-Zusammensetzung weitere Hilfs- und Zusatzstoffe, insbesondere die Folgenden:
- Reaktivverdünner, insbesondere die bereits vorgängig erwähnten, oder epoxidiertes Sojaöl oder Leinöl, Acetoacetatgruppen aufweisende Verbindungen, insbesondere acetoacetylierte Polyole, Butyrolakton, Carbonate, Aldehyde, Isocyanate oder Reaktivgruppen aufweisende Silikone;
- Polymere, insbesondere Polyamide, Polysulfide, Polyvinylformal (PVF), Polyvinylbutyral (PVB), Polyurethane (PUR), Polymere mit Carboxylgruppen, Polyamide, Butadien-Acrylnitril-Copolymere, Styrol-Acrylnitril-Copolymere, Butadien-Styrol-Copolymere, Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere chlorsulfonierte Polyethylene oder Fluor-haltige Polymere oder Sulfonamid-modifizierte Melamine;
- Fasern, insbesondere Glasfasern, Kohlefasern, Cellulosefasern, Metallfasern, Keramikfasern oder Kunststofffasern wie Polyamidfasern oder Polyethylenfasern;
- weitere Feststoffe, insbesondere Holzgranulat oder Zellstoff;
- Nanofüllstoffe, insbesondere Carbon Nanotubes;
- Rheologie-Modifizierer, insbesondere Verdicker oder Antiabsetzmittel;
- Haftverbesserer, insbesondere Organoalkoxysilane;
- flammhemmende Substanzen, insbesondere die bereits genannten Füllstoffe Aluminiumhydroxid oder Magnesiumhydroxid, Antimontrioxid, Antimonpentoxid, Borsäure (B(OH)₃), Zinkborat, Zinkphosphat, Melaminborat, Melamincyanurat, Ammoniumpolyphosphat, Melaminphosphat, Melaminpyrophosphat, polybromierte Diphenyloxide oder Diphenylether, Phosphate wie insbesondere Diphenylkresylphosphat, Resorcinol-bis(diphenylphosphat), Resorcinoldiphosphat-Oligomer, Tetraphenylresorcinoldiphosphit, Ethylendiamindiphosphat, Bisphenol-A-bis(diphenylphosphat), Tris(chloroethyl)phosphat, Tris(chloropropyl)-phosphat, Tris(dichloroisopropyl)phosphat, Tris[3-bromo-2,2-bis(bromomethyl)-propyl]phosphat, Tetrabromo-Bisphenol-A, Bis(2,3-dibromopropylether) von Bisphenol A, bromierte Epoxidharze, Ethylen-bis(tetrabromophthalimid), Ethylen-bis(dibromonorbornandicarboximid), 1,2-Bis(tribromophenoxy)ethan, Tris(2,3-dibromopropyl)isocyanurat, Tribromophenol, Hexabromocyclododecan, Bis(hexachlorocyclopentadieno)cyclooctan oder Chlorparaffine; oder
- weitere Additive, insbesondere dispergiertes Paraffinwachs oder Biozide.

Bevorzugt enthält die Epoxidharz-Zusammensetzung nur einen geringen Gehalt an Verdünnern. Bevorzugt enthält sie weniger als 30 Gewichts-%, besonders bevorzugt weniger als 20 Gewichts-%, insbesondere weniger als 10 Gewichts-%, Verdünner, bezogen auf die gesamte Epoxidharz-Zusammensetzung.

Bevorzugt enthält die Epoxidharz-Zusammensetzung nur einen geringen Gehalt an Wasser, bevorzugt weniger als 5 Gewichts-%, insbesondere weniger als 1 Gewichts-%, Wasser, bezogen auf die gesamte Epoxidharz-Zusammensetzung.

Die Harz- und die Härter-Komponente der Epoxidharz-Zusammensetzung werden in voneinander getrennten Gebinden gelagert. Weitere Bestandteile der Epoxidharz-Zusammensetzung können als Bestandteil der Harz- oder der Härter-Komponente vorhanden sein, wobei gegenüber Epoxidgruppen reaktive weitere Bestandteile bevorzugt ein Bestandteil der Härter-Komponente sind. Ebenfalls möglich ist, dass weitere Bestandteile als eigene, weitere Komponente vorhanden sind.

Ein geeignetes Gebinde zum Lagern der Harz- oder der Härter-Komponente ist insbesondere ein Fass, ein Hobbock, ein Beutel, ein Eimer, eine Büchse, eine Kartusche oder eine Tube. Die Komponenten sind lagerfähig, das heisst, dass sie vor ihrer Anwendung während mehreren Monaten bis zu einem Jahr und länger aufbewahrt werden können, ohne dass sie sich in ihren jeweiligen Eigenschaften in einem für ihren Gebrauch relevanten Ausmass verändern.

Die Harz- und die Härter-Komponente werden kurz vor oder während der Applikation vermischt. Das Mischungsverhältnis wird bevorzugt so gewählt, dass das molare Verhältnis der gegenüber Epoxidgruppen reaktiven Gruppen zu den Epoxidgruppen im Bereich von 0.5 bis 1.5, insbesondere 0.7 bis 1.2, liegt. In Gewichtsteilen liegt das Mischungsverhältnis zwischen der Harz- und der Härter-Komponente typischerweise im Bereich von 1:2 bis 20:1.

Das Mischen der Komponenten erfolgt kontinuierlich oder batchweise mittels eines geeigneten Verfahrens, wobei darauf zu achten ist, dass zwischen dem Mischen der Komponenten und der Applikation nicht zu viel Zeit vergeht und die Applikation innerhalb der Topfzeit erfolgt. Das Mischen und die Applikation können bei Umgebungstemperatur erfolgen, welche typischerweise im Bereich von etwa 5 bis 40 °C, bevorzugt bei etwa 10 bis 35 °C, liegt, oder bei erhöhter Temperatur, insbesondere im Bereich von 40 bis 150 °C, bevorzugt 50 bis 120 °C.

Mit dem Mischen der Komponenten beginnt die Aushärtung der Epoxidharz-Zusammensetzung durch chemische Reaktion. Primäre und sekundäre Aminogruppen und gegebenenfalls vorhandene weitere gegenüber Epoxidgruppen reaktive Gruppen reagieren mit den Epoxidgruppen unter deren Ringöffnung. Als Ergebnis hauptsächlich dieser Reaktionen polymerisiert die Zusammensetzung und härtet dadurch aus.

Die Aushärtung erstreckt sich typischerweise über einige Stunden bis Tage. Die Dauer hängt unter anderem von der Temperatur, der Reaktivität der Bestandteile, deren Stöchiometrie und der Gegenwart bzw. Menge von Beschleunigern ab. Das Amin der Formel (I) ermöglicht eine besonders schnelle Aushärtung, insbesondere auch bei kalten Temperaturen.

Im frisch vermischten Zustand hat die Epoxidharz-Zusammensetzung eine niedrige Viskosität. Bevorzugt liegt die Viskosität 5 Minuten nach dem Mischen der Harz- und der Härter-Komponente bei 20 °C im Bereich von 0.2 bis 20 Pa·s, bevorzugt 0.3 bis 10 Pa·s, insbesondere 0.3 bis 5 Pa·s, gemessen mittels Kegel-Platten Viskosimeter bei einer Schergeschwindigkeit von 10 s⁻¹.

Die Applikation der Epoxidharz-Zusammensetzung erfolgt auf mindestens ein Substrat und/oder in mindestens eine Giessform.

Als Substrat geeignet sind insbesondere:
- Glas, Glaskeramik, Beton, Mörtel, Zementestrich, Faserzement, Backstein, Ziegel, Gips oder Natursteine wie Granit oder Marmor;
- Reparatur- oder Nivelliermassen auf Basis PCC (Polymer-modifizierter Zementmörtel) oder ECC (Epoxidharz-modifizierter Zementmörtel);
- Metalle oder Legierungen wie Aluminium, Eisen, Stahl, Kupfer, weitere Buntmetalle, inklusive oberflächenveredelte Metalle oder Legierungen wie verzinkte oder verchromte Metalle;
- Asphalt oder Bitumen;
- Leder, Textilien, Papier, Holz, mit Harzen, beispielsweise Phenol-, Melamin-oder Epoxidharzen, gebundene Holzwerkstoffe, Harz-Textil-Verbundwerkstoffe oder weitere sogenannte Polymer-Composites;
- Kunststoffe wie Hart- und Weich-PVC, Polycarbonat, Polystyrol, Polyester, Polyamid, PMMA, ABS, SAN, Epoxidharze, Phenolharze, PUR, POM, TPO, PE, PP, EPM oder EPDM, jeweils unbehandelt oder oberflächenbehandelt, beispielsweise mittels Plasma, Corona oder Flammen;
- Faserverstärkte Kunststoffe, wie Kohlefaser-verstärkte Kunststoffe (CFK), Glasfaser-verstärkte Kunststoffe (GFK) und Sheet Moulding Compounds (SMC);
- Isolierschäume, insbesondere aus EPS, XPS, PUR, PIR, Steinwolle, Glaswolle oder geschäumtem Glas (Foamglas);
- beschichtete oder lackierte Substrate, insbesondere lackierte Fliesen, gestrichener Beton, pulverbeschichtete Metalle oder Legierungen oder lackierte Bleche;
- Beschichtungen, Farben oder Lacke, insbesondere beschichtete Böden, welche mit einer weiteren Bodenbelagsschicht überschichtet werden.

Die Substrate können bei Bedarf vor dem Applizieren vorbehandelt werden, insbesondere durch physikalische und/oder chemische Reinigungsverfahren oder das Aufbringen eines Aktivators oder eines Primers.

Die Substrate werden insbesondere beschichtet und/oder verklebt.

Als Giessform geeignet ist eine Vorrichtung, in welche die vermischte, flüssige Epoxidharz-Zusammensetzung gegossen und darin ausgehärtet werden und nach der Aushärtung daraus entformt bzw. entnommen werden kann, wobei die ausgehärtete Zusammensetzung einen Formkörper bildet.

Ein weiterer Gegenstand der Erfindung ist eine ausgehärtete Zusammensetzung erhalten aus der beschriebenen Epoxidharz-Zusammensetzung nach dem Vermischen der Harz- und der Härter-Komponente.

Die Epoxidharz-Zusammensetzung wird bevorzugt verwendet als Beschichtung, Primer, Klebstoff, Dichtstoff, Vergussmasse, Giessharz, Imprägnierharz, oder als Formkörper oder Matrix für Verbundwerkstoffe (Composites) wie insbesondere CFK (enthaltend Carbonfasern) oder GFK (enthaltend Glasfasern) oder Holzverbundwerkstoff.

Ein weiterer Gegenstand der Erfindung ist ein Verfahren zum Beschichten, umfassend die Schritte
(i) Applizieren der vermischten Epoxidharz-Zusammensetzung auf mindestens ein Substrat,
(ii) gegebenenfalls Applizieren einer weiteren Schicht der vermischten Epoxidharz-Beschichtung auf die ausgehärtete Schicht.

Als Substrat geeignet sind die bereits genannten.

Bevorzugt wird die Epoxidharz-Zusammensetzung dabei eingesetzt als Grundierung oder Primer, wobei als Substrat insbesondere Beton, Mörtel, Zementestrich, oder ein Metall, insbesondere Stahl vorhanden ist. Eine solche Epoxidharz-Zusammensetzung ist bevorzugt weitgehend frei von Füllstoffen und dient dazu, das Substrat zu verfestigen, allfällige Poren zu verschliessen und eine gute Haftung zwischen dem Substrat und weiteren Schichten sicherzustellen.

Weiterhin bevorzugt wird die Epoxidharz-Zusammensetzung dabei eingesetzt als Bodenbeschichtung. Eine solche Epoxidharz-Zusammensetzung enthält bevorzugt Füllstoffe und Pigmente und wird in einem oder mehreren Arbeitsgängen insgesamt insbesondere in einer Schichtdicke von 0.1 bis 5 mm bevorzugt als selbstverlaufende Beschichtung flächig appliziert und aushärten lassen. Bevorzugt ist das Substrat dabei ein gegebenenfalls mittels Primer oder Grundierung vorbehandelter Beton, Mörtel, Zementestrich.

Weiterhin bevorzugt wird die Epoxidharz-Beschichtung dabei eingesetzt als Schutzbeschichtung, insbesondere als Korrosionsschutz-Beschichtung auf Stahl oder als Topcoat auf Bodenbeschichtungen. Eine solche Beschichtung wird bevorzugt in einem Spritzverfahren appliziert, insbesondere in einer Schichtdicke von 0.05 bis 0.5 mm.

Ein weiterer Gegenstand der Erfindung ist ein Verfahren zum Verkleben, umfassend das Applizieren der vermischten Epoxidharz-Zusammensetzung,
- entweder auf mindestens eines der zu verklebenden Substrate und Fügen der Substrate zu einer Verklebung,
- oder in einen Hohlraum oder Spalt zwischen mehreren Substraten und gegebenenfalls Einfügen eines Ankers in den Hohlraum oder Spalt.

Als "Anker" wird dabei insbesondere ein Armierungseisen, ein Gewindestab oder ein Bolzen bezeichnet. Ein solcher wird insbesondere so in einer Mauer, Wand, Decke oder in einem Fundament eingeklebt bzw. verankert, dass ein Teil davon kraftschlüssig verklebt ist und ein Teil davon vorsteht und konstruktiv belastet werden kann.

Verklebt werden können gleichartige oder verschiedene Substrate.

Ein weiterer Gegenstand der Erfindung ist ein Verfahren zum Herstellen eines Formkörpers, umfassend das Hineingeben der vermischten Epoxidharz-Zusammensetzung in eine Giessform, gefolgt von der Aushärtung der vermischten Zusammensetzung, gegebenenfalls unter Druck und gegebenenfalls mittels zugeführter Wärme.

Dabei kann die Epoxidharz-Zusammensetzung vor der Aushärtung mit Fasern, Pulvern oder Granulaten versetzt werden, insbesondere mit Carbonfasern, Glasfasern oder Holzpulver und/oder -granulat, um ein Composit-Material zu erhalten. Der Formkörper kann in der Giessform in einem Guss hergestellt werden, oder er kann in Schichten aufgebaut werden.

Nach der Aushärtung wird der Formkörper bevorzugt aus der Giessform entfernt. Er kann weiterverarbeitet werden, insbesondere durch Schneiden, Bohren, Ziehen, Schleifen oder Polieren.

Aus der Verwendung eines Amin der Formel (I) zum Aushärten von Epoxidharzen oder aus einem Verfahren zum Beschichten, zum Verkleben oder zum Herstellen eines Formkörpers entsteht ein Artikel.

Der Artikel ist bevorzugt ein Bauwerk oder ein Teil davon, insbesondere ein Bauwerk des Hoch- oder Tiefbaus, ein Büro, eine Industriehalle, eine Turnhalle, ein Kühlraum, ein Silo, eine Brücke, ein Dach, ein Treppenhaus, ein Fussboden, ein Balkon, eine Terrasse oder ein Parkdeck, oder ein industrielles Gut oder ein Konsumgut, insbesondere eine Lamelle, ein Paneel, ein Pier, eine Offshore-Plattform, ein Schleusentor, ein Kran, eine Spundwand, eine Rohrleitung, ein Gefäss oder ein Rotorblatt einer Windkraftanlage, oder ein Transportmittel wie insbesondere ein Automobil, ein Lastkraftwagen, ein Schienenfahrzeug, ein Schiff, ein Flugzeug oder ein Helikopter, oder ein Anbauteil davon.

Das Amin der Formel (I) ist dabei bevorzugt eingesetzt als Härter, Co-Härter und/oder einbaubaren Beschleuniger.

Ein weiterer Gegenstand der Erfindung ist ein Amin der Formel (la), 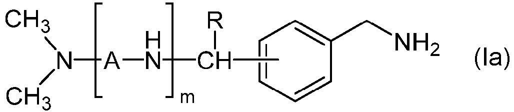 wobei m für 1 oder 2 steht, R für H oder Methyl steht und A für einen Alkylen-Rest mit 2 bis 6 C-Atomen steht.

Bevorzugt steht m für 1.

Bevorzugt steht R für H.

Bevorzugt steht A für 1,2-Ethylen, 1,2-Propylen, 1,3-Propylen, 1,4-Butylen oder 1,5-Pentylen, besonders bevorzugt für 1,2-Ethylen oder 1,3-Propylen, insbesondere für 1,3-Propylen.

Bevorzugt ist das Amin der Formel (la) ausgewählt aus der Gruppe bestehend aus N-(2-Dimethylaminoethyl)-1,3-bis(aminomethyl)benzol, N-(3-Dimethylaminopropyl)-1,3-bis(aminomethyl)benzol, N-(2-Dimethylaminoethyl)-1,4-bis(aminomethyl)-benzol, N-(3-Dimethylaminopropyl)-1,4-bis(aminomethyl)benzol, N-(2-Dimethylaminoethyl)-1,2-bis(aminomethyl)benzol und N-(3-Dimethylaminopropyl)-1,2-bis(aminomethyl)benzol.

Bevorzugt stehen die Substituenten am Benzolring in 1,3- oder in 1,4-Stellung.

Besonders bevorzugt als Amin der Formel (la) ist N-(3-Dimethylaminopropyl)-1,3-bis(aminomethyl)benzol oder N-(3-Dimethylaminopropyl)-1,4-bis(aminomethyl)-benzol.

Ein weiterer Gegenstand der Erfindung ist Reaktionsprodukt enthaltend mindestens ein Amin der Formel (la), erhalten aus der reduktiven Alkylierung von mindestens einem Amin der Formel (IIa) mit mindestens einem Benzonitril der Formel (III) und Wasserstoff, 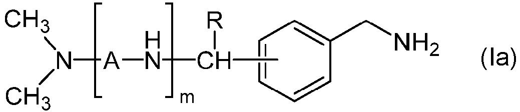 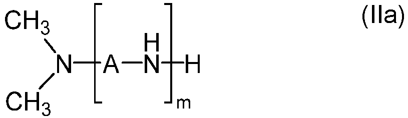 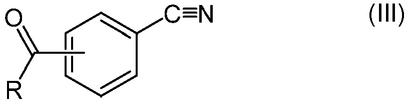 wobei m, R und A die bereits genannten Bedeutungen aufweisen.

Als Amin der Formel (IIa) bevorzugt ist 2-Dimethylaminoethylamin, 3-Dimethyl-amino-2-propylamin, 3-Dimethylaminopropylamin (DMAPA), 4-Dimethylaminobutylamin, 5-Dimethylaminopentylamin oder 3-(3-(Dimethylamino)propylamino)-propylamin (DMAPAPA).

Besonders bevorzugt ist 2-Dimethylaminoethylamin DMAPA, insbesondere DMAPA.

Als Benzonitril der Formel (III) geeignet ist 2-Formylbenzonitril, 3-Formylbenzonitril oder 4-Formylbenzonitril, 2-Acetylbenzonitril, 3-Acetylbenzonitril oder 4-Acetylbenzonitril. Bevorzugt ist 2-Formylbenzonitril, 3-Formylbenzonitril oder 4-Formylbenzonitril, insbesondere 3-Formylbenzonitril oder 4-Formylbenzonitril, am meisten bevorzugt ist 3-Formylbenzonitril.

Das Amin der Formel (II) und das Benzonitril der Formel (III) werden bevorzugt ungefähr stöchiometrisch eingesetzt, insbesondere im Molverhältnis zwischen dem Amin der Formel (II) und dem Benzonitril der Formel (III) von 0.9/1 bis 1.1/1, bevorzugt 0.9/1 bis 1.0/1.

Die reduktive Alkylierung wird wie vorgängig beschrieben durchgeführt.

Das Reaktionsprodukt kann weiter gereinigt werden, insbesondere durch Destillation. Dadurch wird ein Reaktionsprodukt mit einem besonders hohen Gehalt an Amin der Formel (la) erhalten.

Bevorzugt enthält das Reaktionsprodukt einen Gehalt an Amin der Formel (la) von mindestens 70 Gewichts-%, besonders bevorzugt mindestens 80 Gewichts-%, insbesondere mindestens 90 Gewichts-%.

Das Amin der Formel (la) ist geruchsarm und niedrigviskos.

Das Amin der Formel (la), insbesondere in Form des beschriebenen Reaktionsprodukts, wird bevorzugt verwendet zum Aushärten von Epoxidharzen.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

"AHEW" steht für das Aminwasserstoff-Equivalentgewicht.

"EEW" steht für das Epoxid-Equivalentgewicht.

Als "Normklima" ("NK") wird eine Temperatur von 23±1 °C und eine relative Luftfeuchtigkeit von 50±5% bezeichnet.

Die verwendeten Chemikalien waren, sofern nicht anders bezeichnet, von Sigma-Aldrich Chemie GmbH.

### Beschreibung der Messmethoden:

Die **Viskosität** wurde auf einem thermostatisierten Kegel-Platten-Viskosimeter Rheotec RC30 (Kegeldurchmesser 50 mm, Kegelwinkel 1°, Kegelspitze-PlattenAbstand 0.05 mm) gemessen. Niedrigviskose Proben mit einer Viskosität von weniger als 100 mPa·s wurden mit einer Scherrate von 100 s⁻¹ gemessen, höherviskose Proben mit einer Scherrate von 10 s⁻¹.

Die **Aminzahl** wurde durch Titration bestimmt (mit 0.1N HClO₄ in Essigsäure gegen Kristallviolett).

**Gaschromatogramme** (GC) wurden gemessen im Temperaturbereich von 60 bis 320 °C mit einer Aufheizrate von 15 °C/min und 10 min Verweilzeit bei 320 °C. Die Injektortemperatur betrug 250 °C. Es wurde eine Zebron ZB-5 Säule verwendet (L = 30 m, ID = 0.25 mm, dj = 0.5 µm) bei einem Gasfluss von 1.5 ml/Min. Die Detektion erfolgte mittels Flammenionisation (FID).

**Infrarotspektren** (FT-IR) wurden als unverdünnte Filme auf einem mit horizontaler ATR-Messeinheit mit Diamant-Kristall ausgestatteten FT-IR Gerät Nicolet iS5 von Thermo Scientific gemessen. Die Absorptionsbanden sind in Wellenzahlen (cm⁻¹) angegeben.

**¹H-NMR-Spektren** wurden auf einem Spektrometer des Typs Bruker Ascend 400 bei 400.14 MHz gemessen; die chemischen Verschiebungen δ sind angegeben in ppm relativ zu Tetramethylsilan (TMS). Echte und Pseudo-Kopplungsmuster wurden nicht unterschieden.

### Herstellung von Aminen der Formel (I):

### Amin A1: N-(3-Dimethylaminopropyl)-1,3-bis(aminomethyl)benzol

39.2 g (0.30 mol) 3-Formylbenzonitril (= 3-Cyanobenzaldehyd) wurden unter Stickstoffatmosphäre bei 40 °C in 750 ml Isopropylalkohol gelöst, dann langsam unter Rühren mit 32.0 g (0.315 mol) 3-Dimethylaminopropylamin (DMAPA, von Arkema) versetzt und anschliessend 1 Stunden gerührt. Darauf wurde die Reaktionsmischung bei 65 °C, 75 bar Wasserstoff-Druck und einem Fluss von 5 ml/min auf einer kontinuierlich arbeitenden Hydrierapparatur mit Raney-Nickel-Festbettkatalysator hydriert und die hydrierte Lösung am Rotationsverdampfer bei 65 °C eingeengt, wobei unreagiertes 3-Dimethylaminopropylamin, Wasser und Isopropanol entfernt wurden. Erhalten wurden 59.9 g eines gelblichen Öls mit einer Viskosität bei 20 °C von 42 mPa·s.

49.9 g des erhaltenen Öls wurden anschliessend mittels Destillation gereinigt, wobei bei 117 bis 120 °C (Dampftemperatur) und 0.06 mbar 27.3 g Destillat als klares, farbloses Öl mit einer Viskosität bei 20 °C von 28 mPa·s, einer Aminzahl von 732 mg KOH/g und einem mittels GC bestimmten Gehalt an N-Dimethylaminopropyl-1,3-bis(aminomethyl)benzol von > 97 % (Retentionszeit 13.2 min) und einem berechneten AHEW von 73.7 g/eq erhalten wurde, welches im Folgenden als Amin **A1** eingesetzt wurde.

¹H-NMR (CDCl₃): 7.28 (m, 2 H, Ar-H), 7.20 (m, 2 H, Ar-H), 3.85 (d, 2 H, Ar-CH₂-N), 3.79 (s, 2 H, Ar-CH₂-N), 2.68 (t, 2 H, CH₂-N), 2.32 (t, 2 H, CH₂-N(CH₃)), 2.21 (s, 6 H, CH₃), 1.75 (br s, 3 H, NH) , 1.68 (m, 2 H, C-CH₂-C) FT-IR: 3274, 3023, 2936, 2855, 2812, 2763, 1607, 1589, 1457, 1443, 1376, 1261, 1153, 1114, 1040, 837, 780, 738, 698

### Amin A2: N-(3-(Dimethylamino)propylamino)propyl-1,3-bis(aminomethyl)benzol

Das Amin **A2** wurde wie für Amin **A1** beschrieben hergestellt, wobei aber 26.2 g (0.20 mol) 3-Formylbenzonitril (= 3-Cyanobenzaldehyd), 750 ml Isopropanol und 33.4 g (0.21 mol) 3-(3-(Dimethylamino)propylamino)propylamin (DMAPAPA, von Arkema) anstelle von 3-Dimethylaminopropylamin eingesetzt wurden. Erhalten wurden 49.8 g eines gelblichen Öls mit einer Viskosität bei 20 °C von 42 mPa·s, welches mittels Destillation gereinigt wurde.

Bei 148 bis 152 °C (Dampftemperatur) und 0.06 mbar wurden 26.7 g Destillat als klares, farbloses Öl mit einer Viskosität bei 20 °C von 81 mPa·s, einer Aminzahl von 794 mg KOH/g und einem mittels GC bestimmten Gehalt an N-(3-(Dimethyl-amino)propylamino)propyl-1,3-bis(aminomethyl)benzol von > 97 % (Retentionszeit 15.5 min) und einem berechneten AHEW von 69.6 g/eq erhalten, welches im Folgenden als Amin **A2** eingesetzt wurde.

¹H-NMR (CDCl₃): 7.27 (m, 2 H, Ar-H), 7.18 (m, 2 H, Ar-H), 3.85 (d, 2 H, Ar-CH₂-N), 3.77 (s, 2 H, Ar-CH₂-N), 2.69 (m, 6 H, CH₂-N), 2.29 (t, 2 H, CH₂-N(CH₃)), 2.19 (s, 6 H, CH₃) , 1.78 (br s, 4 H, NH) , 1.65 (m, 4 H, C-CH₂-C)

FT-IR: 3294, 3050, 3024, 2912, 2813, 1605, 1588, 1451, 1355, 1336, 1154, 1111, 1027, 890, 779, 732, 696

### Verwendete weitere Substanzen und Abkürzungen:

| | |
|---|---|
| Araldite^{®} GY 250: | Bisphenol A-Diglycidylether, EEW 187 g/Eql (von Huntsman) |
| Araldite^{®} DY-E: | Monoglycidylether von C₁₂- bis C₁₄-Alkoholen, EEW ca. 290 g/Eq (von Huntsman) |
| DMAPAPA: | 3-(3-(Dimethylamino)propylamino)propylamin, AHEW 53 g/Eq (DMAPAPA, von Arkema) |
| B-EDA | N-Benzyl-1,2-ethandiamin, hergestellt wie nachfolgend beschrieben, 150.2 g/mol, AHEW 50.1 g/eq |
| IPDA: | 1-Amino-3-aminomethyl-3,5,5-trimethylcyclohexan, AHEW 42.6 g/Eq (Vestamin^{®} IPD, von Evonik) |
| Ancamine^{®} K54 | 2,4,6-Tris(dimethylaminomethyl)phenol (von Evonik) |

### N-Benzyl-1,2-ethandiamin (B-EDA):

180.3 g (3 mol) 1,2-Ethandiamin wurden bei Raumtemperatur vorgelegt, mit einer Lösung aus 106.0 g (1 mol) Benzaldehyd in 1200 ml Isopropanol vermischt und 2 Stunden gerührt, anschliessend bei 80 °C, 80 bar Wasserstoff-Druck und einem Fluss von 5 ml/min auf einer kontinuierlich arbeitenden Hydrierapparatur mit Pd/C-Festbettkatalysator hydriert und die hydrierte Lösung am Rotationsverdampfer bei 65 °C eingeengt, wobei unreagiertes 1,2-Ethandiamin, Wasser und Isopropanol entfernt wurden. Die so erhaltene Reaktionsmischung wurde bei 80 °C unter Vakuum mittels Destillation gereinigt. Erhalten wurde eine farblose Flüssigkeit mit einem mittels GC bestimmten Gehalt an N-Benzyl-1,2-ethandiamin von > 97%.

### Herstellung von Härtern und Epoxidharz-Zusammensetzungen:

### Beispiele 1 und 2:

Für jedes Beispiel wurden die in Tabelle 1 angegebenen Inhaltsstoffe der Harz-Komponente in den angegebenen Mengen (in Gewichtsteilen) mittels eines Zentrifugalmischers (SpeedMixer^{™} DAC 150, FlackTek Inc.) vermischt und unter Ausschluss von Feuchtigkeit aufbewahrt.

Ebenso wurden die in Tabelle 1 angegebenen Inhaltsstoffe der Härter-Komponente verarbeitet und aufbewahrt.

Anschliessend wurden die beiden Komponenten jeder Zusammensetzung mittels des Zentrifugalmischers zu einer homogenen Flüssigkeit verarbeitet und diese unverzüglich folgendermassen geprüft:
Die **Viskosität** wurde 5 min nach dem Vermischen der Harz- und der Härter-Komponente wie beschrieben bei einer Temperatur von 20 °C gemessen.

Die **Shore D** Härte wurde bestimmt nach DIN 53505 an zwei zylindrischen Prüfkörpern (Durchmesser 20 mm, Dicke 5 mm), wobei einer im Normklima und einer bei 8 °C und 80% relativer Feuchtigkeit gelagert und die Härte jeweils nach 1 Tag (24h) und nach 2 Tagen gemessen wurde.

Weiterhin wurde ein Film in einer Schichtdicke von 500 µm auf eine Glasplatte aufgezogen und dieser im Normklima gelagert bzw. ausgehärtet. An diesem Film wurde die **Königshärte** (Pendelhärte nach König nach DIN EN ISO 1522) nach 1 Tag, 2 Tagen, 7 Tagen und nach 14 Tagen bestimmt **(1d NK), (2d NK), (7d NK), (14d NK).** Nach 14 Tagen wurde der **Aspekt (NK)** des Films beurteilt. Als "schön" wurde ein klarer Film bezeichnet, welcher eine glänzende und nichtklebrige Oberfläche ohne Struktur aufwies. Als "Struktur" wird dabei jegliche Art von Zeichnung oder Muster auf der Oberfläche bezeichnet.

Die Resultate sind in Tabelle 1 angegeben.

**Tabelle 1: Zusammensetzung und Eigenschaften der Beispiele 1 und 2. ¹ nicht messbar (zu weich)**

| **Beispiel** | | **1** | **2** |
|---|---|---|---|
| **Harz-Komponente:** | | | |
| Araldite^{®} GY 250 | | 167.2 | 167.2 |
| Araldite^{®} DY-E | | 31.8 | 31.8 |
| **Härter-Komponente:** | | | |
| **Amin A1** | | 73.7 | - |
| **Amin A2** | | - | 69.6 |
| Viskosität (10') [Pa·s] | | 0.41 | 0.46 |
| Shore D | (1d NK) | 67 | 51 |
| | (2d NK) | 67 | 57 |
| Shore D | (1d 8°/80%) | 45 | n.m.¹ |
| | (2d 8°/80%) | 47 | 46 |
| Königshärte [s] | (1d NK) | 146 | 140 |
| | (2d NK) | 155 | 143 |
| | (7d NK) | 157 | 143 |
| | (14d NK) | 157 | 144 |
| Aspekt (NK) | | schön | schön |

### Beispiele 3 bis 6:

Für jedes Beispiel wurden die in Tabelle 2 angegebenen Inhaltsstoffe der Harz-und der Härter-Komponente wie für Beispiel 1 beschrieben vermischt und die vermischten Komponenten folgendermassen geprüft:
**Viskosität, Shore D, Königshärte (NK)** und **Aspekt (NK)** wurden wie für Beispiel 1 beschrieben bestimmt.

Die **Gelierzeit** wurde bestimmt, indem eine frisch vermischte Menge von ca. 3 g im Normklima mit einem Spatel in regelmässigen Abständen bewegt wurde, bis die Masse gelierte.

Zusätzlich wurde ein weiterer Film in einer Schichtdicke von 500 µm auf eine Glasplatte aufgezogen und dieser unmittelbar nach dem Applizieren während 7 Tagen bei 8 °C und 80% relativer Feuchtigkeit und anschliessend während 2 Wochen im Normklima gelagert, beziehungsweise ausgehärtet. 24 Stunden nach der Applikation wurde ein Flaschendeckel aus Polypropylen auf den Film aufgesetzt, unter welchem ein feuchter Schwamm platziert war. Nach weiteren 24 Stunden wurden der Schwamm und der Deckel entfernt und an einer neuen Stelle des Films platziert, wo sie nach 24 Stunden wieder entfernt und neu platziert wurden, insgesamt 4 mal. Anschliessend wurde der Aspekt dieses Films beurteilt (in den Tabellen mit **"Aspekt (8°/80%)"** bezeichnet), auf die gleiche Weise wie für den Aspekt (NK) beschrieben. Dabei wurde jeweils auch die Anzahl und Art der sichtbaren Marken angegeben, die im Film durch den feuchten Schwamm oder den aufgesetzten Deckel entstanden waren. Als "Blushing" wurde die Anzahl weiss verfärbte Flecken angegeben. Als "(1)" wurde ein schwacher, weiss verfärbter Fleck bezeichnet. Als "1" wurde ein deutlicher, weiss verfärbter Fleck bezeichnet. Als "Ring" wurde angegeben, ob ein ringförmiger Abdruck durch Einsinken des ersten, 24h nach Applikation aufgesetzten Deckels vorhanden war. Ein solcher ringförmiger Abdruck zeigt an, dass die Beschichtung noch nicht begehbar ist. Als "(ja)" wurde ein sehr geringer ringförmiger Abdruck bezeichnet. Als "ja" wurde ein deutlicher ringförmiger Abdruck bezeichnet. An den so ausgehärteten Filmen wurde wiederum die Königshärte bestimmt, jeweils nach 7 Tagen bei 8 °C und 80% relativer Feuchtigkeit **(Königsh. (7d 8°/80%)),** dann nach weiteren 2 Tagen im NK **(Königsh. (+2d NK))** bzw. 7 Tagen im NK **(Königsh. (+7d NK))** bzw. 14d im NK **(Königsh. (+14d NK)).**

Die Resultate sind in Tabelle 2 angegeben.

Vergleichsbeispiele sind mit **"(Ref.)"** bezeichnet.

**Tabelle 2: Zusammensetzung und Eigenschaften der Beispiele 3 bis 6.**

| **Beispiel** | | **3** | **4** | **5 (Ref.)** | **6 (Ref.)** |
|---|---|---|---|---|---|
| **Harz-Komponente:** | | | | | |
| Araldite^{®} GY 250 | | 167.2 | 167.2 | 167.2 | 167.2 |
| Araldite^{®} DY-E | | 31.8 | 31.8 | 31.8 | 31.8 |
| **Härter-Komponente:** | | | | | |
| **Amin A1** | | 22.1 | 11.1 | - | - |
| DMAPAPA | | - | - | 8.0 | - |
| B-EDA | | - | 7.5 | 7.5 | 15.0 |
| IPDA | | 29.8 | 29.8 | 29.8 | 29.8 |
| Benzylalkohol | | 30.0 | 30.0 | 30.0 | 30.0 |
| Salicylsäure | | 2.0 | 2.0 | 2.0 | 2.0 |
| Ancamine^{®} K54 | | - | - | - | 2.0 |
| Viskosität (10') [Pa·s] | | 0.54 | 0.49 | 0.43 | 0.46 |
| Gelierzeit (h:min) | | 3:30 | 4:00 | 4:00 | 4:00 |
| Shore D | (1d NK) | 77 | 71 | 67 | 57 |
| | (2d NK) | 79 | 76 | 73 | 74 |
| Shore D | (1d 8°/80%) | 33 | 13 | 11 | 9 |
| | (2d 8°/80%) | 76 | 69 | 63 | 61 |
| Königshärte [s] | (1d NK) | 105 | 49 | 53 | 34 |
| | (2d NK) | 157 | 119 | 118 | 85 |
| | (7d NK) | 183 | 168 | 163 | 146 |
| | (14d NK) | 185 | 181 | 172 | 158 |
| Aspekt (NK) | | schön | schön | schön | schön |
| Königsh. [s] | (7d 8°/80%) | 105 | 59 | 49 | 32 |
| | (+2d NK) | 164 | 153 | 131 | 106 |
| | (+7d NK) | 175 | 167 | 147 | 137 |
| | (+14d NK) | 183 | 160 | 143 | 137 |
| Aspekt (8°/80%) | | schön | schön | trüb | schön |
| Blushing | | (1) | (1) | 1 | (1) |
| Ring | | (ja) | (ja) | ja | ja |

## Patentansprüche

1. Verwendung eines Amins der Formel (I) zum Aushärten von Epoxidharzen, 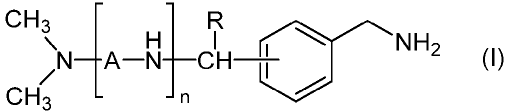 wobei n für 0 oder 1 oder 2 steht, R für H oder Methyl steht und A für einen Alkylen-Rest mit 2 bis 6 C-Atomen steht.

2. Verwendung gemäss Anspruch 1, **dadurch gekennzeichnet, dass** R für H steht.

3. Verwendung gemäss einem der Ansprüche 1 bis 2, **dadurch gekennzeichnet, dass** A für 1,2-Ethylen oder 1,3-Propylen steht.

4. Verwendung gemäss einem der Ansprüche 1 bis 3, **dadurch gekennzeichnet, dass** n für 0 oder 1 steht.

5. Verwendung gemäss einem der Ansprüche 1 bis 4, **dadurch gekennzeichnet, dass** das Amin der Formel (I) ausgewählt ist aus der Gruppe bestehend aus N,N-Dimethyl-1,3-bis(aminomethyl)benzol, N-(2-Dimethylaminoethyl)-1,3-bis-(aminomethyl)benzol, N-(3-Dimethylaminopropyl)-1,3-bis(aminomethyl)benzol, N,N-Dimethyl-1,4-bis(aminomethyl)benzol, N-(2-Dimethylaminoethyl)-1,4-bis-(aminomethyl)benzol, N-(3-Dimethylaminopropyl)-1,4-bis(aminomethyl)benzol, N,N-Dimethyl-1,2-bis(aminomethyl)benzol, N-(2-Dimethylaminoethyl)-1,2-bis-(aminomethyl)benzol und N-(3-Dimethylaminopropyl)-1,2-bis(aminomethyl)-benzol.

6. Verwendung gemäss einem der Ansprüche 1 bis 5, **dadurch gekennzeichnet, dass** das Amin der Formel (I) Bestandteil eines Reaktionsprodukts erhalten aus der reduktiven Alkylierung von mindestens einem Amin der Formel (II) mit mindestens einem Benzonitril der Formel (III) und Wasserstoff ist.

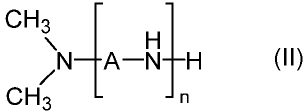

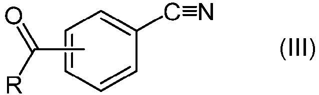

7. Härter für Epoxidharze enthaltend mindestens ein Amin der Formel (I) gemäss einem der Ansprüche 1 bis 6 und mindestens einen weiteren Bestandteil ausgewählt aus weiteren Aminen **A1,** Beschleunigern und Verdünnern.

8. Härter gemäss Anspruch 7, **dadurch gekennzeichnet, dass** mindestens ein weiteres Amin **A1** enthalten ist, wobei das weitere Amin **A1** mindestens drei aliphatische Aminwasserstoffe aufweist.

9. Epoxidharz-Zusammensetzung umfassend
- eine Harz-Komponente enthaltend mindestens ein Epoxidharz und
- eine Härter-Komponente enthaltend mindestens ein Amin der Formel (I) gemäss einem der Ansprüche 1 bis 6 oder einen Härter gemäss einem der Ansprüche 7 oder 8.

10. Ausgehärtete Zusammensetzung erhalten aus der Epoxidharz-Zusammensetzung gemäss Anspruch 9 nach dem Vermischen der Harz- und der Härter-Komponente.

11. Verfahren zum Beschichten, umfassend die Schritte
(i) Applizieren der vermischten Epoxidharz-Zusammensetzung gemäss Anspruch 9 auf mindestens ein Substrat,
(ii) gegebenenfalls Applizieren einer weiteren Schicht der vermischten Epoxidharz-Beschichtung gemäss Anspruch 9 auf die ausgehärtete Schicht.

12. Verfahren zum Verkleben, umfassend das Applizieren der vermischten Epoxidharz-Zusammensetzung gemäss Anspruch 9
- entweder auf mindestens eines der zu verklebenden Substrate und Fügen der Substrate zu einer Verklebung,
- oder in einen Hohlraum oder Spalt zwischen mehreren Substraten und gegebenenfalls Einfügen eines Ankers in den Hohlraum oder Spalt.

13. Verfahren zum Herstellen eines Formkörpers, umfassend das Hineingeben der vermischten Epoxidharz-Zusammensetzung gemäss Anspruch 9 in eine Giessform, gefolgt von der Aushärtung der vermischten Zusammensetzung, gegebenenfalls unter Druck und gegebenenfalls mittels zugeführter Wärme.

14. Amin der Formel (la), 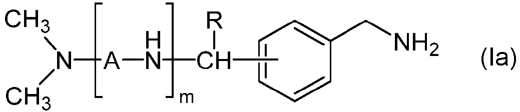 wobei
m für 1 oder 2 steht,
R für H oder Methyl steht, und
A für einen Alkylen-Rest mit 2 bis 6 C-Atomen steht.

15. Reaktionsprodukt enthaltend mindestens ein Amin der Formel (la), erhalten aus der reduktiven Alkylierung von mindestens einem Amin der Formel (IIa) mit mindestens einem Benzonitril der Formel (III) und Wasserstoff, 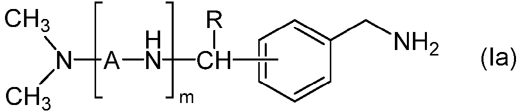 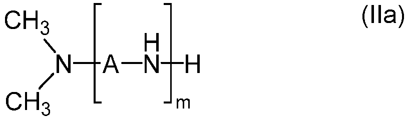 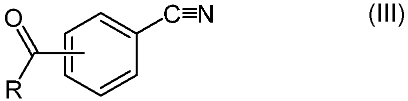 wobei
m für 1 oder 2 steht,
R für H oder Methyl steht, und
A für einen Alkylen-Rest mit 2 bis 6 C-Atomen steht.

## Claims

1. Use of an amine of the formula (I) for curing of epoxy resins, 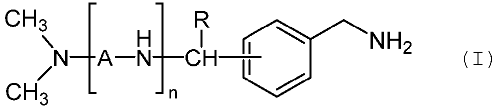 where n is 0 or 1 or 2, R is H or methyl and A is an alkylene radical having 2 to 6 carbon atoms.

2. Use according to Claim 1, **characterized in that** R is H.

3. Use according to either of Claims 1 and 2, **characterized in that** A is 1,2-ethylene or 1,3-propylene.

4. Use according to any of Claims 1 to 3, **characterized in that** n is 0 or 1.

5. Use according to any of Claims 1 to 4, **characterized in that** the amine of the formula (I) is selected from the group consisting of N,N-dimethyl-1,3-bis(aminomethyl)benzene, N-(2-dimethylaminoethyl)-1,3-bis(aminomethyl)benzene, N-(3-dimethylaminopropyl)-1,3-bis(aminomethyl)benzene, N,N-dimethyl-1,4-bis(aminomethyl)benzene, N-(2-dimethylaminoethyl)-1,4-bis(aminomethyl)benzene, N-(3-dimethylaminopropyl)-1,4-bis(aminomethyl)benzene, N,N-dimethyl-1,2-bis(aminomethyl)benzene, N-(2-dimethylaminoethyl)-1,2-bis(aminomethyl)benzene and N-(3-dimethylaminopropyl)-1,2-bis(aminomethyl)benzene.

6. Use according to any of Claims 1 to 5, **characterized in that** the amine of the formula (I) is a constituent of a reaction product obtained from the reductive alkylation of at least one amine of the formula (II) with at least one benzonitrile of the formula (III) and hydrogen.

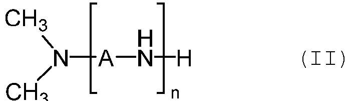

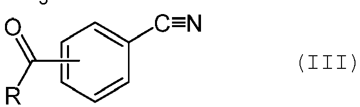

7. Curing agent for epoxy resins comprising at least one amine of the formula (I) according to any of Claims 1 to 6 and at least one further constituent selected from further amines A1, accelerators, and thinners.

8. Curing agent according to Claim 7, **characterized in that** at least one further amine A1 is present, where the further amine A1 has at least three aliphatic amine hydrogens.

9. Epoxy resin composition comprising
- a resin component comprising at least one epoxy resin and
- a curing agent component comprising at least one amine of the formula (I) according to any of Claims 1 to 6 or a curing agent according to either of Claims 7 and 8.

10. Cured composition obtained from the epoxy resin composition according to Claim 9 after the resin component and the curing agent component have been mixed.

11. Method for coating, comprising the steps of
(i) applying the mixed epoxy resin composition according to Claim 9 to at least one substrate,
(ii) optionally applying a further layer of the mixed epoxy resin coating according to Claim 9 to the cured layer.

12. Method of bonding, comprising the applying of the mixed epoxy resin composition according to Claim 9
- either to at least one of the substrates to be bonded and joining the substrates to form a bond,
- or into a cavity or gap between two or more substrates and optionally inserting an anchor into the cavity or gap.

13. Method of producing a shaped body, comprising the introducing of the mixed epoxy resin composition according to Claim 9 into a casting mold, followed by the curing of the mixed composition, optionally under pressure and optionally by means of supplied heat.

14. Amine of the formula (Ia), 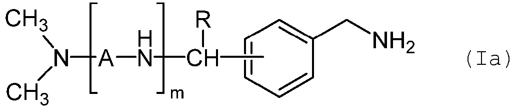 where
m is 1 or 2,
R is H or methyl, and
A is an alkylene radical having 2 to 6 carbon atoms.

15. Reaction product comprising at least one amine of the formula (Ia), obtained from the reductive alkylation of at least one amine of the formula (IIa) with at least one benzonitrile of the formula (III) and hydrogen, 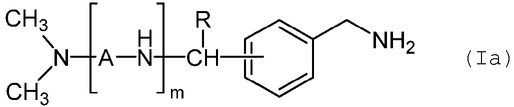 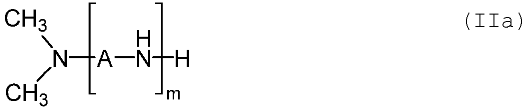 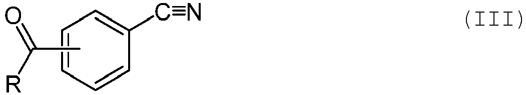 where
m is 1 or 2,
R is H or methyl, and
A is an alkylene radical having 2 to 6 carbon atoms.

## Revendications

1. Utilisation d'une amine de formule (I) pour le durcissement de résines époxyde, 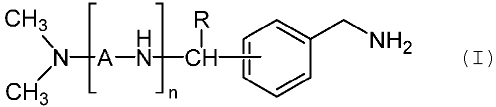 dans laquelle n représente 0 ou 1 ou 2, R représente H ou méthyle et A représente un radical alkylène comprenant 2 à 6 atomes de carbone.

2. Utilisation selon la revendication 1, **caractérisée en ce que** R représente H.

3. Utilisation selon l'une des revendications 1 à 2, **caractérisée en ce que** A représente 1,2-éthylène ou 1,3-propylène.

4. Utilisation selon l'une des revendications 1 à 3, **caractérisée en ce que** n représente 0 ou 1.

5. Utilisation selon l'une des revendications 1 à 4, **caractérisée en ce que** l'amine de formule (I) est choisie dans le groupe constitué par le N,N-diméthyl-1,3-bis(aminométhyl)benzène, le N-(2-diméthylaminoéthyl)-1,3-bis(aminométhyl)benzène, le N-(3-diméthylaminopropyl)-1,3-bis(aminométhyl)benzène, le N,N-diméthyl-1,4-bis(aminométhyl)benzène, le N-(2-diméthylaminoéthyl)-1,4-bis(aminométhyl)benzène, le N-(3-diméthylaminopropyl)-1,4-bis(aminométhyl)benzène, le N,N-diméthyl-1,2-bis(aminométhyl)benzène, le N-(2-diméthylaminoéthyl)-1,2-bis(aminométhyl)benzène et le N-(3-diméthylaminopropyl)-1,2-bis(aminométhyl)benzène.

6. Utilisation selon l'une des revendications 1 à 5, **caractérisée en ce que** l'amine de formule (I) est un constituant d'un produit de réaction obtenu à partir de l'alkylation réductive d'au moins une amine de formule (II) avec au moins un benzonitrile de formule (III) et de l'hydrogène.

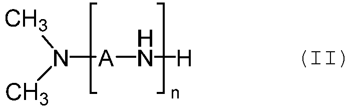

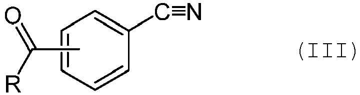

7. Durcisseur pour des résines époxyde, contenant au moins une amine de formule (I) selon l'une des revendications 1 à 6 et au moins un autre constituant choisi parmi d'autres amines A1, les accélérateurs et les diluants.

8. Durcisseur selon la revendication 7, **caractérisé en ce qu'**au moins une autre amine A1 est contenue, l'autre amine A1 présentant au moins trois hydrogènes d'amine aliphatique.

9. Composition de résine époxyde comprenant
- un composant de résine, contenant au moins une résine époxyde et
- un composant durcisseur, contenant au moins une amine de formule (I) selon l'une des revendications 1 à 6 ou un durcisseur selon l'une des revendications 7 à 8.

10. Composition durcie obtenue à partir de la composition de résine époxyde selon la revendication 9 après le mélange des composants de résine et de durcisseur.

11. Procédé pour le revêtement, comprenant les étapes :
(i) application de la composition mélangée de résine époxyde selon la revendication 9 sur au moins un substrat,
(ii) le cas échéant application d'une autre couche du revêtement mélangé de résine époxyde selon la revendication 9 sur la couche durcie.

12. Procédé de collage, comprenant l'application de la composition mélangée de résine époxyde selon la revendication 9
- soit sur au moins l'un des substrats à coller et assemblage des substrats pour un collage,
- soit dans un espace creux ou dans une fente entre plusieurs substrats et le cas échéant insertion d'un ancrage dans l'espace creux ou la fente.

13. Procédé de fabrication d'un corps façonné, comprenant l'introduction de la composition mélangée de résine époxyde selon la revendication 9 dans un moule de coulée, suivie du durcissement de la composition mélangée, le cas échéant sous pression et le cas échéant au moyen de chaleur alimentée.

14. Amine de formule (Ia), 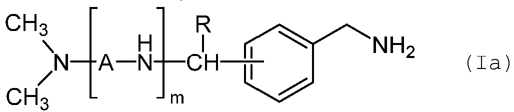 dans laquelle
m représente 1 ou 2,
R représente hydrogène ou méthyle et
A représente un radical alkylène comprenant 2 à 6 atomes de carbone.

15. Produit de réaction contenant au moins une amine de formule (Ia), obtenue à partir de l'alkylation réductive d'au moins une amine de formule (IIa) avec au moins un benzonitrile de formule (III) et de l'hydrogène, 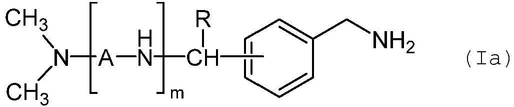 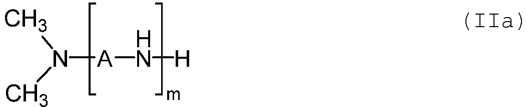 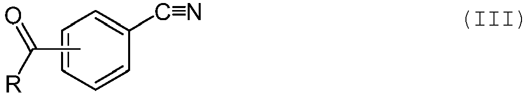 dans lesquelles
m représente 1 ou 2,
R représente hydrogène ou méthyle et
A représente un radical alkylène comprenant 2 à 6 atomes de carbone.
